# EUROPEAN PATENT SPECIFICATION

(11) **EP 0 923 952 B1**
(45) Date of publication and mention of the grant of the patent: **17.09.2003**
(21) Application number: 97120946.5
(22) Date of filing: 28.11.1997
(51) Int. Cl.: A61L 9/01

(54) **Enhanced nitrate deodorant composition**
Desodorierende Nitratzusammensetzung
Composition désodorisante à base de nitrate

(43) Date of publication of application: 23.06.1999
(73) Proprietor: Thetford Corporation, Ann Arbor, Michigan 48103 (US)
(72) Inventor: van Nijnatten, Hans A.M., 4824 RS Breda (NL); Van der Pluijm, Frans A., 5103 BT Dongen (NL)
(74) Representative: Wössner, Gottfried, Dr. Dipl.-Chem.

(56) References cited:
- WO-A-97/06834
- DE-A- 4 328 372
- US-A- 5 609 863

## Description

The present invention relates generally to a deodorant composition and more particularly, to a deodorant composition comprising nitrate and a preservative.

### BACKGROUND OF THE INVENTION

Deodorants for mobile sanitary equipment or other sewage-related systems are frequently based on one or more biocides also known as preservatives. These preservatives are used in an attempt to kill or at least inhibit the fecal bacteria and thus prevent microbial activity and associated stench. Microbial activity results in oxygen consumption and eventually to anaerobic conditions. By anaerobic is meant no oxygen available in any form; no dissolved oxygen gas, no nitrates and/or sulfates. Under anaerobic conditions, mercaptans (fecal odor corresponding with rotten cabbage) and later, hydrogen sulfide (decay odor resembling rotten eggs) are released. These gasses are not only very unpleasant but also toxic, even in low concentrations.

It is practically impossible to kill all microorganisms in a sewage holding tank. One reason Is that human feces contain a large amount of bacteria both aerobic and anaerobic (10.2 E10 per gram, Hill et al 1971). Feces slowly dissolve in water and will keep re-infecting the water for a long period of time. Other factors that inhibit anti-microbial action are the high organic load and the presence of counter-acting molecules like ammonia. The concentration of biocides used by leading companies is therefore well above the minimum inhibitory concentration for fecal bacteria (see Table 1). Traditionally, formaldehyde has been used as a preservative and as an odor neutralizer. Alternatively, quaternary ammonium salts such as Bardac 22 (dimethyldidecyl ammonium chloride) or Arquad CB50 (benzalkonium chloride) are used as well as other preservatives such as Myacide AS (2-bromo-2-nitropropanediol, Tris nitro and others. These preservatives are also widely used to prevent product spoilage in consumer products and in disinfectant surface cleaners.

There is substantial evidence that many if not all of these frequently used preservatives are easily and fully broken down In the environment especially in a sewage treatment plant environment. Sewage treatment plant microorganisms are specialized to degrade complex organic molecules including preservatives. Unfortunately, concentration is a very important factor for the biodegradability of preservatives and, in order for sewage organisms to degrade the preservatives, the concentration of the preservatives must be below the minimum Inhibitory concentration level.

A "non-biocidal" way to prevent odors is to delay the system from getting anaerobic by providing an additional oxygen source, generally nitrate. Nitrates are widely used in sewage systems to prevent odors and to reduce or prevent the presence of toxic gases like hydrogen sulfide. Hydrogen sulfide is released if bacteria use oxygen from sulfates. If the system becomes anoxic (dissolved oxygen gas used up) bacteria use nitrate as a source of oxygen. If nitrates are used up, bacteria use sulfates resulting in the production of hydrogen sulfide. The specific bacterial flora, temperature, organic load and the time before a tank is emptied, influence the speed in which the nitrate is used up and thus the overall performance of the deodorant.

WO-A- 9 706 834 discloses a dry nitrate deodorant composition. DE-A-4 328 372 discloses the use of nitrate with a preservative and optionally alcohols for odor control.

It would thus be desirable to provide a deodorant composition which minimizes the concentration of preservative and therefore maximizes biodegradability, without compromising on performance.

### SUMMARY OF THE INVENTION

A deodorant composition is provided which may be used to reduce odorous gases in sewage-related settings such as waste holding tanks. The composition of the present invention comprises nitrate in combination with a preservative as defined in claim 1 wherein the concentration of the preservative is lower than the concentration commonly used in toilet deodorants, with surprisingly good deodorant performance. By utilizing a concentration of preservative which is below the minimum inhibitory concentration level, the preservative is more biodegradable by specialized sewage microorganisms, compared to commercially available deodorant compositions having higher preservative concentrations. The nitrate component of the present invention further enhances the performance of the composition by supplying an additional oxygen source for the bacteria present In the sewage, thereby decreasing the production of hydrogen sulfide caused by bacterial consumption of sulfates.

Methods of making and using the compositions of the present invention are also provided as defined in claims 7 and 13 respectively.

Additional objects, advantages, and features of the present invention will become apparent from the following description and appended claims, taken in conjunction with the accompanying drawings.

### BRIEF DESCRIPTION OF THE DRAWINGS

The various advantages of the present invention will become apparent to one skilled in the art by reading the following specification and subjoined claims and by referencing the following drawings in which:
Figure 1 is a schematic representation of a device used In the testing of the compositions of the present invention;
Figure 2 is a graph showing the results of testing of a composition of the present invention measured with a Multi-Rea® device;
Figure 3 is a graph showing the results of testing of a composition of the present invention measured with a Jenway® 9200, dissolved oxygen meter; and
Figure 4 is a graph showing the effects of varying the preservative component of the composition.

### DETAILED DESCRIPTION OF THE PREFERRED EMBODIMENTS

The deodorant compositions of the present invention comprise nitrate in combination with a preservative as defined in claim 1, wherein the concentration of the preservative is lower than the concentration commonly used in toilet deodorants, with surprisingly good deodorant performance. By utilizing a lower concentration of preservative, the preservative is more biodegradable by sewage microorganisms, compared to commercially available deodorant compositions having higher preservative concentrations. The nitrate component ofthe present invention further enhances the performance of the composition by supplying an additional oxygen source for the bacteria present in the sewage, thereby decreasing the production of hydrogen sulfide caused by bacterial consumption of sulfates. In addition to nitrate and preservative, the compositions of the present invention may also comprise a surfactant, a fragrance, a dye and a thickener. A stabilizer in the form of a glycol (ethylene- or propyleneglycol) or a carboxylic acid may also be used to enhance the stability of the formulation.

The nitrate component of the compositions of the present invention are those known to those skilled In the art and are generally water-soluble nitrate salts, including, but not limited to calcium, sodium, ammonium and potassium. Calcium nitrate available from van Epenhuyzen is preferred. The concentration of nitrate is from 1% to 40% by weight of the total composition, wherein from about 10% to about 20% is preferred.

The preservative component ofthe compositions of the present invention may be chosen from those normally used In the art or used as active ingredients in disinfectant cleaners. Examples Include Bardac 22 available from Lonza, Myacyde AS from Knoll, Arquad CB50 by AKZO and Tris-nitro from Angus. A preservative concentration of 0.3% to 15% by weight of the total composition, preferably from about 1% to about 7.5%, used. More specifically, Myacide AS may be used at a concentration of from about 0.5% to about 3% by weight of the total composition, preferably from about 1% to about 2%. For quaternary ammonium salts, from 0.3 % to about 5%, and preferably from about 0.5% to about 2% may be used. For Tris-nifro, from about 1% to about 12%, preferably between about 4% to about 10% may be employed. It will be appreciated that preferred percentage ranges are based on commonly used dosages of 50-150 ml per 10 liter holding tank capacity.

Those skilled in the art will recognize that there are hundreds of biocides/preservatives (used interchangeably herein) that may be employed in the compositions of the present invention, especially those which perform in environments with a massive number of bacteria, a high organic load and the presence of ammonia. Any suitable biocide/preservative may therefore be employed, wherein the concentration is at a level where the biocide/preservative is biodegradable by sewage microorganisms such as alcohols as 2-bromo-2-nitro-propanediol (Myacide AS), formaldehyde donors as 2-hydroxymethyl-2-nitro-1,3-propanediol (Tris-nitro) and quaternary ammonium salts like alky-benzyl-dimethyl-ammoniumchloride (Arquad CB50).
It will be appreciated that a combination of biocides/preservatives can also be used.

The fragrance employed in the present invention may be any fragrance composition commonly used in the art. For example Citrus Super Strong 212063 available from Drom may be employed, and Pinolin F532.393 available from Quest International is preferred. Also chemicals known in the art as "malodor counteractants" can be used instead of a fragrance. It will be appreciated that the amount of fragrance used will be dependent on the type of fragrance composition employed, however, from about 0.5% to about 5% by weight of the total composition is generally sufficient and from about 0.5% to about 2% is preferred. While not wishing to be bound by theory, it is believed that the fragrance masks any odors produced by the anaerobic bacteria.

In an alternative embodiment of the present invention, a surfactant may also be used to emulsify the fragrance. Without wishing to be bound by theory, it is believed that the surfactant aids in digesting waste, thereby making it more accessible to the nitrate and preservative. The surfactant may be anionic or cationic and nonionic surfactants are preferred such as Tween 20 or cremofor from Bayer and Emulgin RT40, available from Henkel. The amount of surfactant employed is from about 0.1% to about 5% by weight of the total composition, wherein from about 0.5% to about 1.5% is preferred.

In an alternative embodiment of the present invention, a thickener can be used and will generally be based on the type of preservatives used. For example, Natrosol HHR available from Hercules can be used if the preservative employed is a quaternary ammonium compound and a Xanthan Gum type like Rodopol G from Rhone Poulenc or Keltrol RD from Kelco if other preservatives are used. Furthermore a solvent can be used to stabilize the formulation. Generally mono propylene glycol (MPG) or carboxylic acids may be used in a concentration of from about 0.2% to about 10% by weight of the total composition. Dye, such as a non-staining type like Blue JMT available from Miliken, may also be employed.

Those skilled in the art will readily recognize that the present Invention may be employed in various sewage-related settings *i.e*., where fluid waste is not being drained directly into a sewage system. The present invention is particularly useful for waste holding tanks, for example in portable and mobile lavatories and recreational vehicles. Those skilled in the art will also appreciate that an effective amount of the composition of the present invention may be determined based on the knowledge of one so skilled and the specific application.

The following specific examples further describe the present invention.

### SPECIFIC EXAMPLE 1

A particular formulation for a chemical toilet was prepared containing the following ingredients:

| | |
|---|---|
| Calcium nitrate (50%) | 30% |
| 2-bromo-2-nitropropanediol (BNPD) | 1.5% |
| Fragrance Pinolin | 0.8% |
| Surfactant Emulgin RT40 | 0.75% |
| Bleu dye | 0.57% |
| Thickener Keltrol RD | 0.3% |
| Mono propylene glycol | 1% |
| Acetic acid (10%) | 0.2% |
| Soft water | balance |

A premix was made by adding/mixing Fragrance Pinolin, Surfactant Emulgin RT40, Thickener Keltrol RD and Mono propylene glycol. In another beaker the water phase acetic acid, soft water and 2-bromo-2-nitropropanediol were mixed until homogenous. Then the premix was added to the water phase and mixed for at least 15 minutes. At that time calcium nitrate (50%) was added and after Quality Control Bleu dye was added to complete the formulation.

Table 1 shows the concentration of preservative commonly used compared to the concentration of preservative employed in the compositions of the present invention.

**Table 1**

| **Frequently used preservatives In high performance toilet- deodorants*** | **% in exemplary composition of the present invention** | **advised dosage in 20 liter tank** | **cone. in 20 liter tank (ppm)** | **MIC in ppm**** |
|---|---|---|---|---|
| formaldehyde | 24 | 120 | 1500 | 31.25 |
| benzalkonium chloride | 9.5 | 150 | 705 | 10 |
| dimethyldidecylammonium chloride | 1.27 | 260 | 165 | 5 |
| 2-bromo-2-nitropropanediol | 4 | 120 | 240 | 31.25 |

| | | | | |
|---|---|---|---|---|
| *Internal reports | | | | |
| **Wallhauzer 1995 MIC *E. coli* | | | | |

### SPECIFIC EXAMPLE 2

The formulation set forth in Specific Example 1 was tested In a Thetford Porta Potti® portable toilet. A dosage of 120 ml of the toilet deodorant set forth in Specific Example 1 was used. The holding tank was heated for 8 hours a day with a heating pad to simulate summer conditions. The toilet was used for four days by adult males for both urinations and defecations. The average odor grade of 3 tests during use, given by a six person trained odor panel, at the end of the use period, was 2.7 on a 5 point scale, wherein:

| |
|---|
| 1 = pleasant |
| 2 = OK |
| 3 = acceptable |
| 4 = tolerable |
| 5 = not tolerable |

The average odor grade given by a seven person odor panel when the toilet was emptied was 2.8 on the same 5 point scale.

### SPECIFIC EXAMPLE 3

Another particular formulation was prepared containing the following ingredients:

| | |
|---|---|
| Calcium nitrate (50%) | 30% |
| 2-hydroxymethyl-2-nitro-1,3-propanediol | 6% |
| Fragrance Pinolin | 0.8% |
| Surfactant Emulgin RT40 | 0.56% |
| Bleu dye | 0.64% |
| Thickener Keltrol RD | 0.3% |
| Mono propylene glycol | 1% |
| Soft water | balance |

A premix was made by adding mixing Fragrance Pinolin, Surfactant Emulgin RT40, Thickener Keltrol RD and Mono propylene glycol. In another beaker the water phase, soft water and 2-hydroxymethyl-2-nitro-1,3-propanediol were mixed until homogenous. Then the premix was added to the water phase and mixed for at least 15 minutes. At that time calcium nitrate (50%) was added and after Quality Control Bleu dye was added to complete the formulation.

This formulation was used two times in the same test as described in Specific Example 2. The results for two tests were an odor grade of 2.7 during use and a 3.3 grade when the toilet was emptied.

### SPECIFIC EXAMPLE 4

Another particular formulation was prepared containing the following ingredients:

| | |
|---|---|
| Calcium nitrate (50%) | 30% |
| Arquad CB50 | 2.5% |
| Fragrance Pinolin | 0.8% |
| Sodiumbicarbonate | 0.66% |
| Bleu dye | 0.57% |
| Natrasol 250 HHR (Hercules/Aqualon) | 0.5% |
| Akypo LF4 (carboxylic acid from Kao) | 3% |
| Surfactant Emulgin RT40 | 0.75% |
| Soft water | balance |

A premix was made by adding/mixing Arquad CB50, Fragrance Pinolin and Surfactant Emulgin RT40. In another beaker the water phase, soft water, sodiumbicarbonate and Natrasol 250 HHR (Hercules/Aqualon) were mixed until homogenous. Then Akypo LF4 (carboxylic acid from Kao) was added to the water phase and mixed for at least 15 minutes and then the premix was added and mixed for 5 minutes. At that time calcium nitrate (50%) was added to complete the formulation.

### SPECIFIC EXAMPLE 5

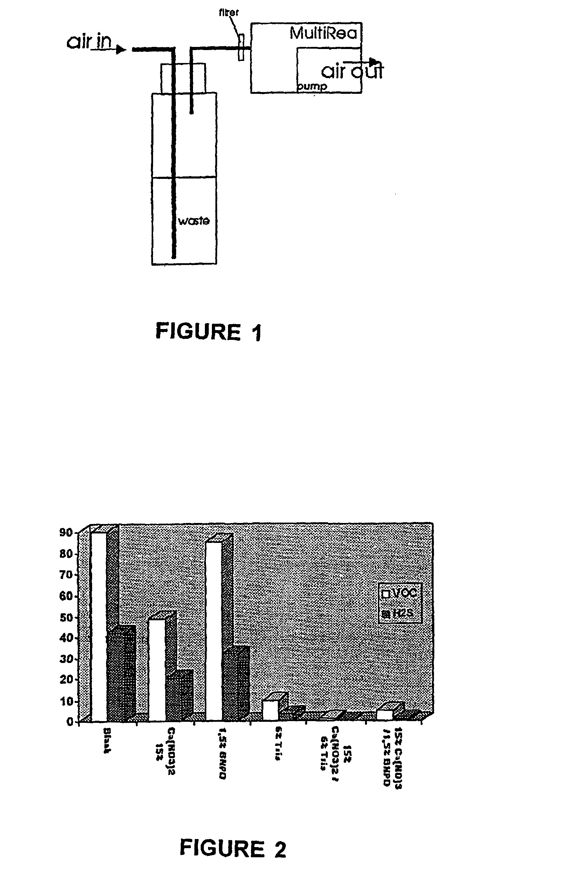

Several combinations (watery solutions) of preservatives and nitrate were used in an attempt to deodorize (10%) a human waste solution under controlled conditions. Mixing 1-part human feces with 1.2 parts of fresh urine made the waste. 100 grams of the waste was mixed with 900 grams of tap water to gen 1000 grams of (10%) waste solution. To 100 ml of this waste, 0.3 ml of several chemicals were added and poured in a 200 ml gas wash bottle (Figure 1).

After incubation at 29° C for 48 hours headspace gasses were measured. At the time of the measurement the average gas temperature was 24/25° C. The gases were pumped from the headspace through a MultiRea® device. In this device the hydrogen sulphide concentration is measured with an accurate electrochemical cell and mercaptans, expressed as VOC (volatile organic carbons), with a photo ionization detector. The correction factor for mercaptans is 0.6 (or 60%), the calibration gas was isobutylene.

Figure 2 shows the results. Because the headspace contains a mixture of gasses no correction factor is used in the graphs, raw data are expressed. The results of an Hydrogen sulphide reduction Is most important because these results are the most specific. Data show some (non linear) relationship of the concentration.

The effect of combinations is remarkably and unexpectedly good when compared with the effect of single components. One skilled in the art will appreciate that specific results will depend on the specific feces and urine (waste) combination and other factors like incubation time and temperature. Similar effects were observed with several different types of waste (male, female and child).

### SPECIFIC EXAMPLE 6

In an equivalent test as Specific Example 2, the concentration of dissolved oxygen was also measured with a Jenway® 9200, dissolved oxygen meter. Formaldehyde was used as the standard biocide. In the blank, no chemicals were added.

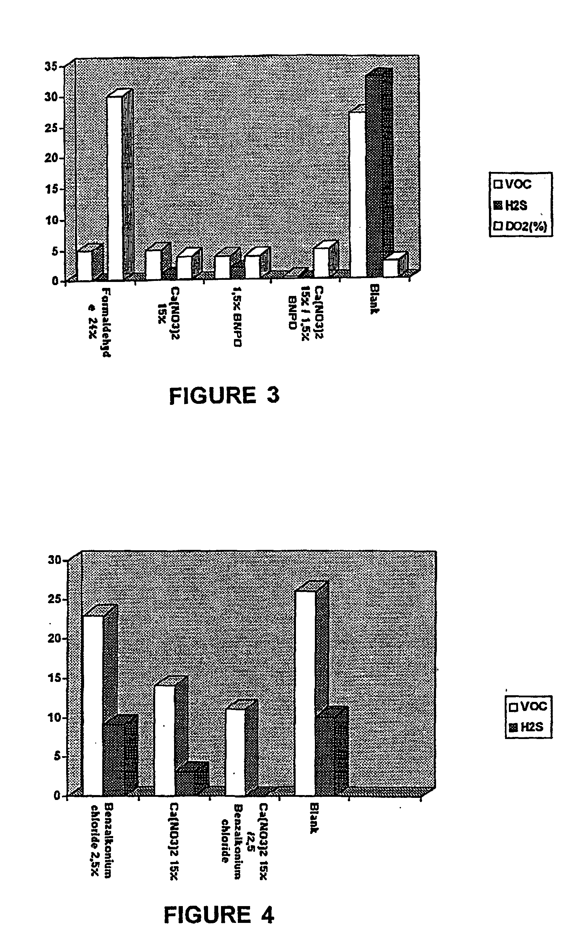

As shown in Figure 3, in the presence of effective concentrations of preservative and nitrate, the respiration is not significantly inhibited. It is recognized in the art that respiration is one of the most important factors in sewage treatment processes and biodegradability.

### SPECIFIC EXAMPLE 7

In a similar test as Specific Example 2, a watery solution of a quaternary ammonium salt (Arquad CB50) was used singly and in combination with calcium nitrate.

As shown in Figure 4, although the combination is clearly better than the single ingredient, the obtained effect depends on the particular type of preservative employed. Results may also vary depending on the type of waste, incubation time and temperature.

## Claims

1. A liquid deodorant composition comprising a solvent, nitrate and a preservative, wherein the preservative constitutes from about 0.3% to about 15% by weight of the total composition, wherein the preservative is chosen from the group consisting of alcohols, formaldehyde donors and quaternary ammonium salts and wherein the nitrate constitutes from about 1% to about 40% by weight of the total composition.

2. The composition of Claim 1, wherein the nitrate is calcium nitrate.

3. The composition of Claim 1, wherein the composition further comprises a surfactant.

4. The composition of Claim 1, wherein the composition further comprises a fragrance.

5. The composition of Claim 1, wherein the composition further comprises a thickener.

6. The composition of Claim 1, wherein the composition further comprises a dye.

7. A method of making a liquid deodorant composition comprising mixing a solvent, nitrate and a preservative, wherein the preservative constitutes from about 0.3% to about 15% by weight of the total composition, wherein the preservative is chosen from the group consisting of alcohols, formaldehyde donors and quaternary ammonium salts and wherein the nitrate constitutes from about 1% to about 40% by weight of the total composition.

8. The method of Claim 7, wherein the nitrate is calcium nitrate.

9. The method of Claim 7, wherein the composition further comprises a surfactant.

10. The method of Claim 7, wherein the composition further comprises a fragrance.

11. The method of Claim 7, wherein the composition further comprises a thickener.

12. The method of Claim 7, wherein the composition further comprises a dye.

13. A method of deodorizing a waste holding tank comprising the steps of:
a) providing a liquid deodorant composition comprising a solvent, nitrate and a preservative, wherein the preservative constitutes from about 0.3% to about 15% by weight of the total composition, wherein the preservative is chosen from the group consisting of alcohols, formaldehyde donors and quaternary ammonium salts and wherein the nitrate constitutes from about 1% to about 40% by weight of the total composition;
b) applying the composition of a) to the waste holding tank.

14. The method of Claim 13, wherein the nitrate is calcium nitrate.

15. The method of Claim 13, wherein the composition further comprises a surfactant.

16. The method of Claim 13, wherein the composition further comprises a fragrance.

17. The method of Claim 13, wherein the composition further comprises a thickener.

18. The method of Claim 13, wherein the composition further comprises a dye.

## Patentansprüche

1. Flüssige Deodorant-Zusammensetzung, umfassend ein Lösemittel, Nitrat und ein Konservierungsmittel, worin das Konservierungsmittel ca. 0,3 bis ca. 15 Gew.% der Gesamtzusammensetzung darstellt, worin das Konservierungsmittel ausgewählt ist aus der Gruppe, welche aus Alkoholen, Formaldehyd-Donoren und quaternären Ammoniumsalzen besteht, und worin das Nitrat ca. 1 bis ca. 40 Gew.% der Gesamtzusammensetzung darstellt.

2. Zusammensetzung nach Anspruch 1, worin das Nitrat Calciumnitrat ist.

3. Zusammensetzung nach Anspruch 1, worin die Zusammensetzung ferner ein Tensid umfasst.

4. Zusammensetzung nach Anspruch 1, worin die Zusammensetzung ferner einen Duftstoff umfasst.

5. Zusammensetzung nach Anspruch 1, worin die Zusammensetzung ferner ein Verdickungsmittel umfasst.

6. Zusammensetzung nach Anspruch 1, worin die Zusammensetzung ferner einen Farbstoff umfasst.

7. Verfahren zur Herstellung einer flüssigen Deodorant-Zusammensetzung, umfassend das Mischen eines Lösemittels, Nitrats und eines Konservierungsmittels, worin das Konservierungsmittel ca. 0,3 bis ca. 15 Gew.% der Gesamtzusammensetzung ausmacht, worin das Konservierungsmittel ausgewählt ist aus der Gruppe, welche aus Alkoholen, Formaldehyd-Donoren und quaternären Ammoniumsalzen besteht, und worin das Nitrat ca. 1 bis ca. 40 Gew.% der Gesamtzusammensetzung ausmacht.

8. Verfahren nach Anspruch 7, worin das Nitrat Calciumnitrat ist.

9. Verfahren nach Anspruch 7, worin die Zusammensetzung ferner ein Tensid umfasst.

10. Verfahren nach Anspruch 7, worin die Zusammensetzung ferner einen Duftstoff umfasst.

11. Verfahren nach Anspruch 7, worin die Zusammensetzung ferner ein Verdickungsmittel umfasst.

12. Verfahren nach Anspruch 7, worin die Zusammensetzung ferner einen Farbstoff umfasst.

13. Verfahren zum Deodorieren eines Schmutzwasserhaltetanks, umfassend die Schritte von:
a) Bereitstellen einer flüssigen Deodorant-Zusammensetzung, umfassend ein Lösemittel, Nitrat und ein Konservierungsmittel, worin das Konservierungsmittel ca. 0,3 bis ca. 15 Gew.% der Gesamtzusammensetzung ausmacht, worin das Konservierungsmittel ausgewählt ist aus der Gruppe, welche aus Alkoholen, Formaldehyd-Donoren und quaternären Ammoniumsalzen besteht, und worin das Nitrat ca. 1 bis ca. 40 Gew.% der Gesamtzusammensetzung ausmacht;
b) Anwenden der Zusammensetzung von Schritt a) auf den Schmutzwasserhaltetank.

14. Verfahren nach Anspruch 13, worin das Nitrat Calciumnitrat ist.

15. Verfahren nach Anspruch 13, worin die Zusammensetzung ferner ein Tensid umfasst.

16. Verfahren nach Anspruch 13, worin die Zusammensetzung ferner einen Duftstoff umfasst.

17. Verfahren nach Anspruch 13, worin die Zusammensetzung ferner ein Verdickungsmittel umfasst.

18. Verfahren nach Anspruch 13, worin die Zusammensetzung ferner einen Farbstoff umfasst.

## Revendications

1. Composition désodorisante liquide comprenant un solvant, du nitrate et un préservateur, dans laquelle le préservateur constitue d'environ 0,3 % à environ 15 % en poids de la composition totale, dans laquelle le préservateur est choisi dans le groupe constitué d'alcools, de donneurs de formaldéhyde et de sels d'ammonium quaternaire et dans laquelle le nitrate constitue d'environ 1 % à environ 40 % en poids de la composition totale.

2. Composition de la revendication 1, dans laquelle le nitrate est le nitrate de calcium.

3. Composition de la revendication 1, dans laquelle la composition comprend en plus un tensioactif.

4. Composition de la revendication 1, dans laquelle la composition comprend en plus un parfum.

5. Composition de la revendication 1, dans laquelle la composition comprend en plus un épaississant.

6. Composition de la revendication 1, dans laquelle la composition comprend en plus un colorant.

7. Procédé de fabrication d'une composition désodorisante liquide comprenant de mélanger un solvant, du nitrate et un préservateur, dans lequel le préservateur constitue d'environ 0,3 % à environ 15 % en poids de la composition totale, dans lequel le préservateur est choisi dans le groupe constitué d'alcools, de donneurs de formaldéhyde et de sels d'ammonium quaternaire et dans lequel le nitrate constitue d'environ 1 % à environ 40 % en poids de la composition totale.

8. Procédé de la revendication 7, dans lequel le nitrate est le nitrate de calcium.

9. Procédé de la revendication 7, dans lequel la composition comprend en plus un tensioactif.

10. Procédé de la revendication 7, dans lequel la composition comprend en plus un parfum.

11. Procédé de la revendication 7, dans lequel la composition comprend en plus un épaississant.

12. Procédé de la revendication 7, dans lequel la composition comprend en plus un colorant.

13. Procédé de désodorisation d'une cuve contenant des eaux usées comprenant les étapes consistant à ;
(a) fournir une composition désodorisante liquide comprenant un solvant, du nitrate et un préservateur, dans laquelle le préservateur constitue d'environ 0,3 % à environ 15 % en poids de la composition totale, dans laquelle le préservateur est choisi dans le groupe constitué d'alcools, de donneurs de formaldéhyde et de sels d'ammonium quaternaire et dans laquelle le nitrate constitue d'environ 1 % à environ 40 % en poids de la composition totale ;
(b) appliquer la composition de a) à la cuve contenant des eaux usées.

14. Procédé de la revendication 13, dans lequel le nitrate est le nitrate de calcium.

15. Procédé de la revendication 13, dans lequel la composition comprend en plus un tensioactif.

16. Procédé de la revendication 13, dans lequel la composition comprend en plus un parfum.

17. Procédé de la revendication 13, dans lequel la composition comprend en plus un épaississant.

18. Procédé de la revendication 13, dans lequel la composition comprend en plus un colorant.
